# EUROPEAN PATENT SPECIFICATION

(11) **EP 2 056 751 B1**
(45) Date of publication and mention of the grant of the patent: **20.04.2011**
(21) Application number: 07841109.7
(22) Date of filing: 20.08.2007
(51) Int. Cl.: A61F 2/90

(54) **INTRAVASCULAR STENT**
INTRAVASKULÄRER STENT
STENT INTRAVASCULAIRE

(30) Priority: 22.08.2006 US 823239 P; 22.08.2006 US 507852
(43) Date of publication of application: 13.05.2009
(60) Divisional application: 11157372.1
(73) Proprietor: Abbott Cardiovascular Systems Inc., Santa Clara, CA 95054 (US)
(72) Inventor: TA, Diem Uyen, San Jose, California 95120 (US); CHI, David, San Francisco, California 94122 (US); ATLADOTTIR, Svava Maria, San Francisco, California 94107 (US); SVENSSON, Bjorn, Gilroy, California 95020 (US)
(74) Representative: McLeish, Nicholas Alistair Maxwell
(86) International application number: PCT/US2007/076315
(87) International publication number: WO 2008/024712

(56) References cited:
- EP-A- 1 236 445
- EP-A- 1 277 449
- WO-A-00/62710
- WO-A-02/15824
- WO-A-98/22159
- WO-A-99/15108
- DE-A1- 19 913 978
- US-A1- 2002 058 989
- US-A1- 2004 127 977
- US-A1- 2004 230 293
- US-A1- 2005 043 782
- US-A1- 2006 036 312
- US-A1- 2006 100 695
- US-B1- 6 805 898
- US-B1- 7 090 694

## Description

### BACKGROUND OF THE INVENTION

The invention relates to vascular repair devices and, in particular, to intravascular stents including rings with sub-structure reducing strain and enhancing flexibility as well as to stents having non-directional structural patterns.

Stents are generally tubular-shaped devices which function to hold open a segment of a blood vessel. They also are suitable for use to support and hold back a dissected arterial lining that can occlude the fluid passageway. That is, whether self-expanding or expandable using force, stents are delivered within vasculature in a radially compressed configuration and then implanted at an interventional site while assuming a radially expanded configuration. At present, there are numerous commercial stents being marketed throughout the world. For example, some known prior art stents have multiplex cylindrical rings connected by one or more links. While some of these stents are flexible and have the appropriate radial rigidity needed to hold open a blood vessel, there typically is a tradeoff between flexibility and radial strength and the ability to tightly compress or crimp the stent onto a catheter so that it does not move relatively to the catheter or dislodge prematurely prior to controlled implantation at an interventional site.

Known stents include that described in US 2005/0043782 in which there is described an expandable stent for implanting in a body lumen, such as a coronary artery, peripheral artery, or other body lumen. The intravascular stent has a plurality of cylindrical rings connected by undulating links. The stent has a high degree of flexibility in the longitudinal direction, yet has adequate vessel wall coverage and radial strength sufficient to hold open an artery or other body lumen. The stent can be compressed or crimped onto a catheter to a very low profile since the peaks that are adjacent the curved portion of the undulating link are shorter than other peaks in the same cylindrical ring to prevent overlap yet still achieve a very low profile, tightly crimped stent onto a catheter.

Similarly, US 200410230293 describes an intravascular stent assembly for implantation in a body vessel, such as a coronary artery, which includes undulating circumferential rings having peaks on the proximal end and valleys on the distal end. Adjacent rings are coupled together by links. The rings and links are arranged so that the stent has good conformability as it traverses through, or is deployed in, a tortuous body lumen. The stent is also configured such that the likelihood of peaks and valleys on adjacent rings which point directly at each other to overlap in tortuous body vessels is reduced.

As stated, various conventional stents include a plurality of rings connected by links. In certain stents, the rings include a plurality of peaks and valleys connected by bar arms. When these rings are positioned in phase relatively to one another, W-crests and Y-crests are formed at the points of connection between the links and rings. Once a stent embodying this structure is implanted at an interventional site, a significant amount of strain is placed upon the peaks and valleys. In fact, the link can become angulated or twisted upon stent expansion resulting in an overall twisted stent configuration. Such a twisted stent configuration can suffer from inadequate vessel wall apposition and thus, may not perform optimally in holding a vessel open. Further, the degree of twisting often cannot be predicted due to manufacturing and material variability which consequently limits the reliability of stent function.

Other factors also contribute to the unpredictability of stent performance. That is, conventional stents embody a pattern of links and rings which can be characterized as directional in configuration. A typical stent can include a pattern of adjacently arranged rings which extend the length of a stent and includes a first end which differs from that of a second end of the stent. Due to this directional structure, such a stent must be placed upon a catheter in a particular direction so that when it is deployed and implanted within vasculature, the stent will be arranged as contemplated to achieve expected performance. Unfortunately, conventional stents embodying directional structure can be placed on a catheter incorrectly due to operator error, and it is difficult to either identify this error or to correct it during a surgical procedure.

Moreover, conventional stents are typically designed to have a relatively uniform flexibility across the length of the stent. However, this uniform flexibility is often not optimal for moving the stent through curved portions of the body. Accordingly, what has been needed and heretofore unavailable is a stent that is particularly more flexible at one or both ends than the center portion of the stent for better movement through the body.

Also, conventional stents tend to have a uniform drug coating. This can be problematic when, for example, the physician overlaps stents. Accordingly, what has been needed and heretofore unavailable is a stent that has greater drug coating on the center portion of the stent as compared to one or both ends of the stent. It can be particularly desirable to combine this non-uniform drug coating with a stent having superior flexibility at one or both ends, as described above.

Further, conventional stents tend to be uniformly radiopaque. However, this uniformity can present problems when, for example, a physician overlaps stents and needs to better monitor the overlapped regions of the stent. Consequently, what has been needed and heretofore unavailable is a stent that has enhanced radiopaque properties at one or both ends of the stent, as compared to the center portion of the stent.

Accordingly, what has been needed and heretofore unavailable is a stent including structure which provides desired flexibility without compromising radial strength and reduces unwanted stresses and twisting. Moreover, there is a need for a stent which addresses problems associated with directional stents as well as stent performance within the body. The present invention satisfies these and other needs.

### SUMMARY OF THE INVENTION

The present invention is directed to an intravascular stent including structure enhancing flexibility without compromising radial strength and minimizing twisting and inherent stresses. The present invention is also directed towards a stent incorporating a non-directional pattern of rings and links.

The present invention provides a flexible intravascular stent having the features set out in claim 1.

Thus, in one aspect, the invention includes a flexible intravascular stent for use in a body lumen, comprising a plurality of cylindrical rings aligned along a common longitudinal axis and interconnected to form the stent, each cylindrical ring having a first delivery diameter and a second larger implanted diameter. Each cylindrical ring includes an open W or butterfly pattern to which one end of a link between adjacent rings is connected. Further, each ring includes a plurality of first peaks, second peaks, and third peaks, adjacent third peaks defining the butterfly pattern. Each of the peaks has a height and an apex, the first peaks being taller than the second peaks, and the second peaks being taller than the third peaks. Described in another way, each cylindrical ring has a plurality of first valleys, second valleys, and third valleys, adjacent third valleys forming the butterfly pattern. Moreover, each of the valleys has a depth and an apex, the first valleys being deeper than the second valleys, and the second valleys being deeper than the third valleys.

At least one link attaches each cylindrical ring to an adjacent cylindrical ring. The links can include an undulation having a curved portion extending transverse to the stent longitudinal axis toward the second peak. Additionally, the curved portion of the undulating link can be longitudinally aligned with the second peak. Also, each undulating link can include an arm that is straight and parallel to the longitudinal axis of the stent and which is circumferentially offset from the second peak. In one particular embodiment, the first peaks are connected to the undulating link and configured adjacent the W or butterfly pattern to relieve stress in a manner to minimize flaring of the ends of the stent.

The invention further contemplates peaks and valleys having a different radii and/or heights than other or adjacent peaks and valleys. In still another aspect of the invention, at least a portion of the links or cylindrical rings can have a variable thickness configuration and/or a variable width.

Still another aspect of the invention is directed towards a non-directional stent. Such a stent is not required to be mounted onto a stent delivery system in a particular proximal-distal orientation. The configuration of peaks and valleys at a proximal end of the stent is generally a mirror image of the configuration of peaks and valleys at the distal end. Both the proximal end and the distal end cylindrical rings may include various combinations of tall peaks, intermediate peaks, short peaks, deep valleys, intermediate valleys, and shallow valleys. Further, the non-directional stent includes the open W or butterfly pattern. In one embodiment, at least two of the open W or butterfly pattern configurations face in opposite directions from each other along the length of the stent. In addition, it is contemplated that not all of the curved portions of the undulating links face in the same direction in the non-directional stent. Because the proximal and distal ends of the stent are generally mirror image configurations or a rotated mirror image of the opposite end of the stent, and the configuration of peaks and valleys of the rings is reversed at one or more points along the length of the stent, the non-directional stent may be mounted onto a stent delivery system in either direction.

The present invention is also directed to an intravascular stent with customized flexibility, drug coating and/or radiopacity properties. In one embodiment, the present invention is directed towards a stent with proximal and/or distal ends that are more flexible than the center portion of the stent. This design may also have a drug coating, in which the drug coating is greater at the center of the stent than at the ends. One or more ends of the stent may be more radiopaque than the center portion of the stent.

Considering one aspect, the invention includes a stent in which one or both ends are more flexible than the center portion, and a drug coating that is greater on the center portion than at the ends. Consequently, a flexible intravascular stent for use in a body lumen has a plurality of cylindrical rings aligned along a common longitudinal axis. At least one link connects adjacent cylindrical rings. The stent has a first end portion, a center portion, and a second end portion, with at least one of the end portions being more flexible than the center portion. The stent has a drug coating, the drug coating having greater coverage on the center portion than on at least one of the end portions.

This embodiment may have one or more of the following aspects. Both ends may be more flexible than the center portion. At least one connecting link in one or both of the end portions may be very flexible with, for example, a shape that increases flexibility, such as a shape with multiple turns. At least one or more cylindrical rings in at least one end portion comprises peaks of uniform extent. One or more cylindrical rings in at least one end portion may have members with a "U" or "Y" profile. At least one link in the center portion may have a "U" profile. At least one cylindrical ring in the center portion may have at least one members with a butterfly pattern.

According to another aspect of the invention, a stent may have one end that is more flexible than the rest of the stent. This will typically be the distal end of the stent which, when flexing through a curve in the body, is the leading edge of the stent and after which the rest of the stent will follow. Such a stent has a plurality of cylindrical rings aligned along a common longitudinal axis. At least one link connects adjacent cylindrical rings. The stent has a first end portion, a center portion, and a second end portion. At least one of the end portions is more flexible than the center portion.

According to another aspect of the invention, a stent has at least one end that is more radiopaque than the center portion. Such a stent has a plurality of cylindrical rings aligned along a common longitudinal axis. At least one link connects adjacent cylindrical rings. The stent has a first end portion, a center portion, and a second end portion. At least one of the end portions is more radiopaque than the center portion.

This type of stent may have a design with greater surface area density at one or both of the end portions than at the center portion. Consequently, for a metal stent, an end portion that has the greater surface area density will be more radiopaque than the center portion.

The present invention therefore provides the stent designer with options for customizing a stent design so as to meet design requirements for a given environment within the body and/or for particular patient requirements.

Other features and advantages of the present invention will become more apparent from the following detailed description of the invention, when taken in conjunction with the accompanying exemplary drawings.

### BRIEF DESCRIPTION OF THE DRAWINGS

The invention is herein described, by way of example only, with reference to the accompanying drawings, wherein:
FIG. 1 is a plan view of a portion of a flattened stent of one embodiment of the invention which illustrates a pattern of rings and links.
FIG. 2A is a plan view of a portion of the stent of FIG. 1 forming a radially compressed cylindrical configuration.
FIG. 2B is a perspective view of a portion of the stent of FIG. 2A.
FIG. 3A is a plan view of a portion of the stent of FIG. 1 in a flattened configuration and illustrating the rings and links in an expanded configuration.
FIG. 3B is a perspective view of a portion of the stent of FIG. 1 in a cylindrical configuration and illustrating the rings and links in an expanded configuration.
FIG. 4 is an enlarged plan view of a portion of the stent of FIG. 1.
FIG. 5 is an enlarged plan view of a portion of an embodiment of a stent including straight links in en expanded configuration.
FIG. 6 is an enlarged cutaway perspective view of a portion of an embodiment of a stent having stents with varying radial thickness.
FIG. 7 is an enlarged plan view of a portion of the stent of FIG. 1.
FIG. 8 is a plan view of a portion of a flattened stent of another pattern of rings and links.
FIG. 9A is a plan view of a portion of one embodiment of a non-directional stent in a flattened configuration.
FIG. 9B is a perspective view of a portion of the stent of FIG. 9A in a cylindrical configuration.
FIG. 10A is a plan view of a portion of the stent of FIG. 9A in a flattened compressed configuration.
FIG. 10B is a perspective view of a portion of the stent of FIG. 10A forming a radially compressed cylindrical configuration.
FIG. 11 is a plan view of a portion of another design of a non-directional stent in a flattened configuration.
FIG. 12 illustrates properties of a stent having end regions having greater flexibility than the center, and a center region that has more drug coating than the ends.
FIG. 13 is a plan view of a portion of a flattened stent having the properties of FIG. 12 and that illustrates a pattern of rings and links.
FIG. 14 is a perspective view of the stent of FIG. 13 as it appears deployed in a straight portion of the body.
FIG. 15 is a perspective view of the stent of FIG. 13 as it appears deployed in a curved section of the body.
FIG. 16 illustrates properties of a stent having a single end that is more flexible than the rest of the stent.
FIG. 17 illustrates properties of a stent having ends that are more radiopaque than the center portions of the stent.

### DETAILED DESCRIPTION OF THE PREFERRED EMBODIMENTS

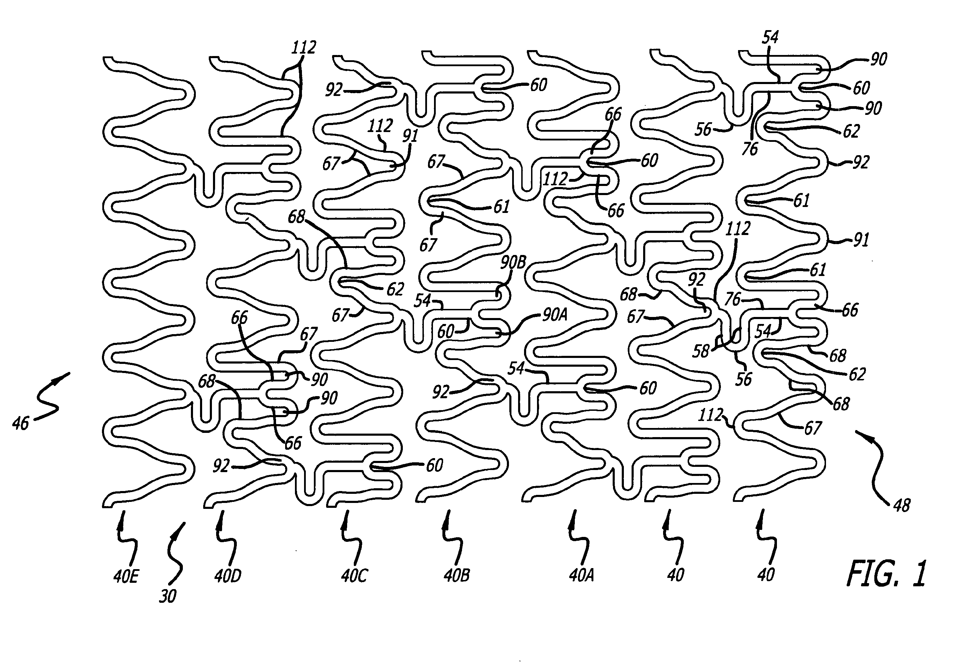

Referring to the drawings, which are provided for purposes of illustration and by way of example but not limitation, the present invention is illustrated in FIGS. 1-7.

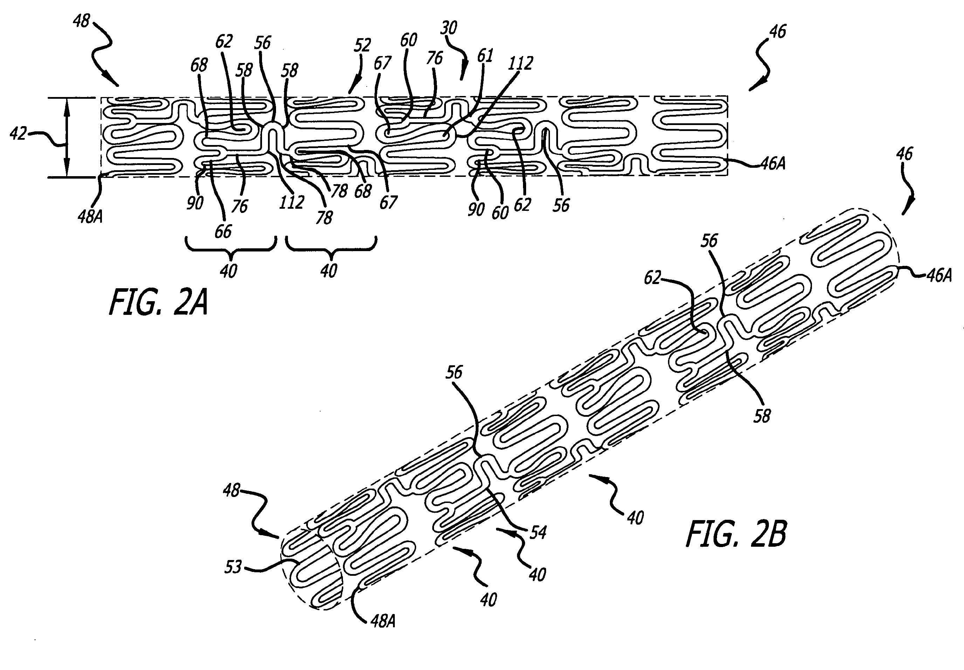

Turning now to FIG. 1, an exemplary stent 30 of the present invention is shown in a flattened condition so that the pattern can be clearly viewed, even though the stent is in a cylindrical form in use, such as shown in FIGS. 2A-B. The stent is typically formed from a tubular member, however, it can be formed from a flat sheet such as shown in FIG. 1 and rolled into a cylindrical configuration.

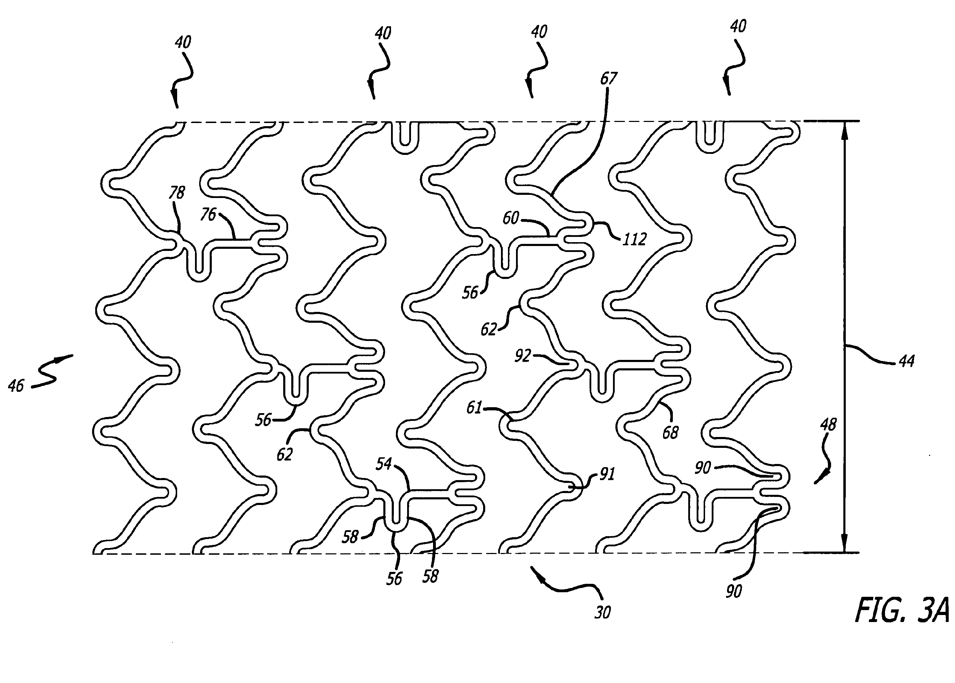

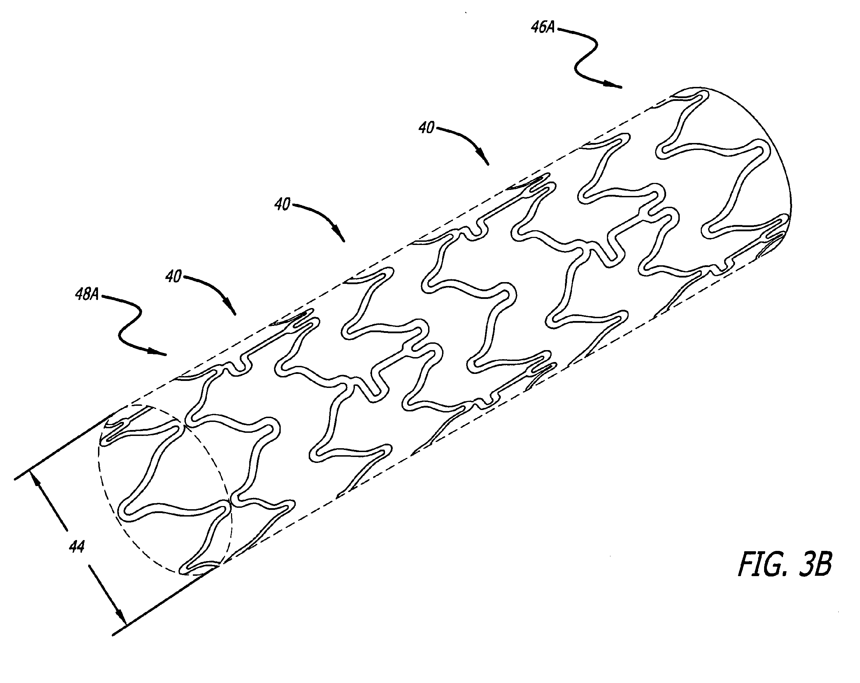

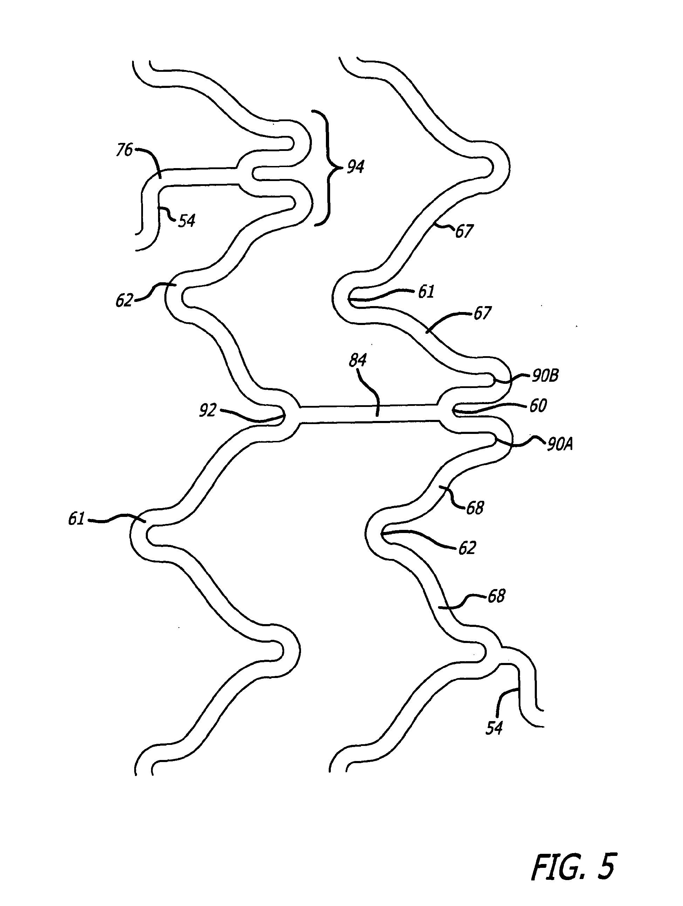

As shown in FIGS. 1-3B, stent 30 is made up of a plurality of cylindrical rings 40 which extend circumferentially around the stent. It is to be recognized that there may be fewer or more cylindrical rings than is shown in the illustrated drawings. The rings are aligned along a common longitudinal axis and interconnected by links 54 to form the stent. The links 54 can be generally straight members (See FIG. 5) or can include one or more curves or turns as shown in the figures. Moreover, links with undulation can be configured at all locations or any selected locations along the stent. The stent has a delivery diameter 42 (FIG. 2A), and expands to an implanted diameter 44 (FIGS. 3A and 3B). The stent has a proximal end 46 and a distal end 48. Typically, since the stent is contemplated to be laser cut from a tube, there are no discreet parts.

Referring specifically to FIG. 2B, each cylindrical ring includes a cylindrical outer wall surface 52 which defines the outermost surface of the stent and a cylindrical inner wall surface 53 which defines the innermost surface of the stent. The links 54 connect one cylindrical ring 40 to an adjacent cylindrical ring 40. To prevent links 54 from compromising the longitudinal flexibility of the stent, a curved portion 56 is incorporated into link 54. This curved portion 56 is connected to one or more substantially straight portions 58 wherein the straight portions 58 are substantially perpendicular to the longitudinal axis of the stent. Thus, as the stent is being delivered through a tortuous vessel, such as a coronary artery, the curved portions 56 and straight portions 58 of the undulating links will permit the stent to flex in the longitudinal direction which substantially enhances delivery af the stent to the target site. The number of bends and straight portions in a link can be increased or decreased from that shown to achieve different flexibility constructions. In one embodiment (not shown), it is contemplated that the links can comprise a plurality of curved portions and straight portions 58. With the straight portions being substantially perpendicular to the stent longitudinal axis, the link 54 acts much like a hinge at the curved portion 56 to provide flexibility in both crimped and expanded stales. The number of links 54 can be adjusted to vary the longitudinal flexibility in the crimped and expanded states of a stent a plurality of

The stent 30 further can be described as having rings including a plurality of alternative peaks and valleys. The peaks and valleys are of different sizes. The rings include one or more open W or butterfly patterns of struts to which links between adjacent rings are connected. Accordingly, the rings include a plurality of short peaks 60, tall peaks 61, intermediate peaks 62, shallow valleys 90, deep valleys 91, and intermediate valleys 92. The peaks and valleys are formed from various length struts and apices 112. In one embodiment, the struts include short struts 66, long struts 67, and intermediate struts 68. The lengths of these struts can be varied to achieve a desired expansion diameter. As shown, each open W or butterfly pattern is defined by one short peak 60, two short struts 66, one long strut 67 and one intermediate strut 68. The struts can be either curved or straight depending upon a particular application. Also, as shown, each peak has a height, the tall peaks being taller than the intermediate peaks, and the intermediate peaks being taller than the short peaks. Additionally, each valley has a depth, the deep valleys being deeper than the intermediate valleys, and the intermediate valleys being deeper than the shallow valleys. In other embodiments, a greater range in the types of heights of peaks and/or a greater range in the types of depths of valleys may be included.

Additionally, in one aspect, the stent 30 can further include one or more Y patterns of struts. With reference to FIG. 2B, the Y pattern is defined by one intermediate valley 92, one long strut 67, and one intermediate strut 68.

It is also contemplated that a stent of the present invention can further include at least one additional peak (not shown) having a different height than the short peak, the tall peak, and the intermediate peak. Also, the stent can further include at least one additional valley (not shown) having a different depth than the shallow valley, the intermediate valley, and the deep valley. For example, one embodiment may have four different height peaks and four different depth valleys. Moreover, the number of peaks and valleys can vary in number for each ring depending upon the application. Thus, for example, if the stent is to be implanted in a coronary artery, a lesser number of peaks and valleys are required than if the stent is implanted in a peripheral artery, which has a larger diameter than a coronary artery. Moreover, the number of peaks and valleys can be adjusted to vary the expansion diameter. Furthermore, the order or juxta-positioning of various sized peaks and valleys can be varied within a ring or from one ring to another, and the various peaks and valleys of adjacent rings can be aligned or offset from each other. It is to be understood that the definition of an open W pattern should not be limited but generally should imply the presence of a plurality of apexes including both one or more peaks and valleys of different heights and depths,

In one particular embodiment, illustrated as example in the peaks 60, 61 and 62 of each ring 40 are oriented towards the proximal end 46, and the valleys 90, 91 and 92 of each ring 40 are oriented towards the distal end 48. These rings can be positioned in phase relatively to one another, meaning that the peaks of one ring are separated from the peaks of the adjacent ring by one ring width plus the spacing between the rings. Likewise, the valleys of one ring are separated from the valleys of the adjacent ring by one ring width plus the spacing between the rings.

As stated, it may be desirable under certain circumstances to position the peaks so that they are out of phase (not shown), that is, the apexes of the peaks of one ring are circumferentially offset from the apexes of the peaks of an adjacent ring. Positioning the peaks, valleys, and links in this manner, provides a stent having desirable expansion capabilities, high radial strength, a high degree of flexibility, and sufficient wall coverage to support a vessel.

As can be seen, for example, in FIGS. 1-2B, curved portion 56, straight portions 58 have been designed such that when crimped, intermediate peak 62 would nest in the space just distal to the curved portion 56 and straight portions 58. This nesting allows the stent 30 to be tightly crimped onto a delivery system to achieve a low crimped OD.

Referring to FIGS. 2A-2B, the crimping or compressing process, circumferentially moves the undulating link 54 along with its curved portion 56 closer to the intermediate peak 62. Although the various stent struts, curved portions, links, and peaks and valleys may contact each other when the stent is crimped or compressed, it may be desirable to avoid the overlapping of struts 66, 67, 68, apexes 112, and links 54.

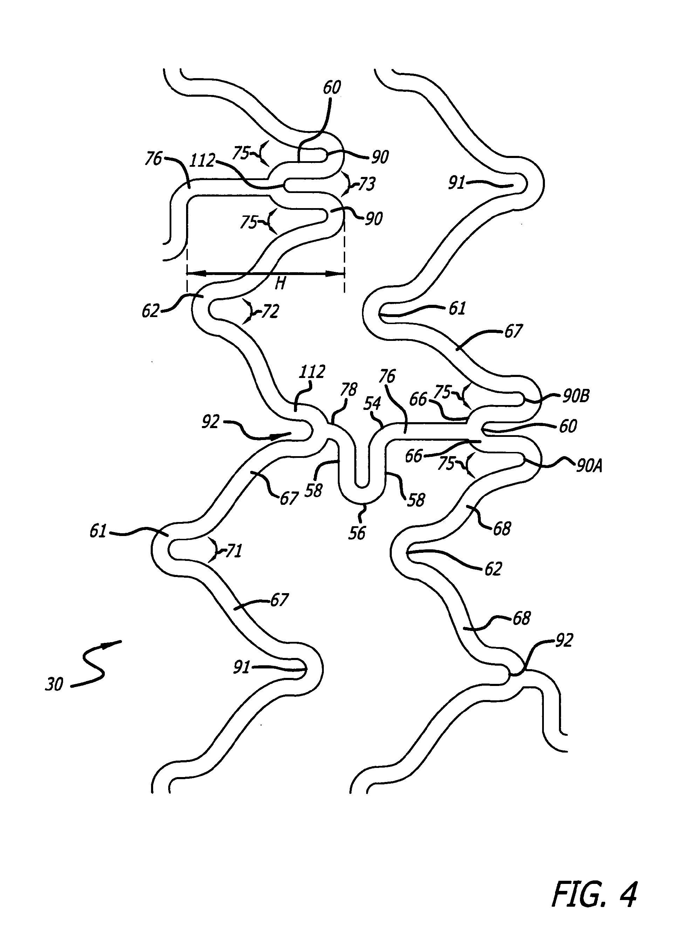

Referring now to FIG. 4, in one embodiment, an arm 76 of the link 54 is attached to the apex 112 of a short peak 60. The length of the arm may vary in different embodiments. The other end 78 of the link 54 is attached to the apex 112 of an intermediate valley 92. Notably, in this embodiment, combined height "H" of the short peak 60 and the arm 76 is longer than the length of the intermediate peak 62 when the stent is in both the compressed and expanded configurations. This allows the stent to be tightly compressed onto a catheter or other stent delivery device, and such structure can be employed to avoid overlapping between the undulating link 54 and the intermediate peak 62. In addition, the circumferential positioning of the intermediate peak 62 and tall peaks 61 can be varied to avoid the intermediate peak 62 and tall peak 61 from touching arm 78 of link 54.

Due to the intricate patterns as disclosed in FIGS. 1-4, the rate of expansion of the various portions of the stent can vary. Accordingly, one aspect of the invention provides for different radii of curvature at various apexes 112 so that the stent will expand evenly and uniformly. Referring more specifically now to FIG. 4, first radius 71 which corresponds with tall peak 61 may have a smaller radius of curvature than does second radius 72 which corresponds with intermediate peak 62. Generally, the longer the struts associated with a peak, the more easily that portion of the stent will expand, so that a smaller radius is associated with peaks having two long struts 67. Likewise, for peaks, such as short peak 60, which has struts 66 that are shorter than the struts 67 of tall peak 61, the apex 112 may have a greater radius 73 of curvature which will expand more easily in order to compensate for the stiffer bending moments created by the shorter struts 66. In yet other embodiments, the radii of curvature of the various peaks and various valleys may be adjusted so that the different types of peaks and valleys expand at different tensions rather than expanding uniformly. In addition, the circumferential positioning of the intermediate peak 62 and tall peaks 61 can be varied to achieve uniform expansion.

The radii 75 of the shallow valleys 90 may also be varied to provide uniform stent expansion. Since a shallow valley formed by an intermediate strut 68 and a short strut 66 can have a tendency to expand more slowly as the stent is expanded compared to a shallow valley formed by a long strut 67 and a short strut 66, a greater radius of a curvature may be incorporated into the shallow valley having the intermediate strut 68. Thus, third radius 75 of a first shallow valley 90A may be greater than the fourth radius 75 of a second adjacent shallow valley 90B. By varying the radii of curvature in the shallow valleys, the stent may expand more evenly and compensate for the varying rates of expansion of adjacent portions in a cylindrical ring.

Typical stents known in the art undergo a lot of strain as they go from a compressed configuration to an expanded configuration. The strain produced by the expansion of a stent may cause the links to be angulated, resulting in a twisted stent. With reference to FIG. 5, the open W butterfly pattern 94 is shown including two shallow valleys 90 connected with each other by a short peak 60. The butterfly pattern 94 is designed to reduce the strain exerted on the peaks 60, 61, 62, valleys 90, 91, 92, struts 66, 67, 68, and links 54 during expansion of the stent 30. Moreover, the butterfly pattern 94 design facilitates achieving better crimping profiles since the short crest and long crest are positioned further away from the linear link, so during crimping the long crest and short crest have a longer distance to travel before hitting the linear link. It is especially beneficial when the stent is coated with a drug because it prevents or at least minimizes a possibility of coating damage. Moreover, the butterfly W crest increases the stent flexibility since both valleys 90 are two separate components which can move to accommodate any bending when the crimped stent tracks through tortuousity.

As previously staled, it is also a design feature that more or fewer links 54 including curved portions be positioned between adjacent cylindrical rings 40. As shown in FIG. 5, straight links 84 in addition to undulating links 54 may be included to connect adjacent cylindrical rings. The straight links can be employed to provide stability and assist in stent length change, as an adjunct to the undulating links.

Further, the straight links may be employed in various alternative approaches to provide more rigidity in a localized area, such as at the stent ends. For example, it may be desirable to incorporate more straight links between the cylindrical rings at the stent ends 46, 48 (FIG. 2B) than in the interposed rings 40A, 40B, 40C (FIG. 1) of the stent. In fact, in one contemplated embodiment, all links can assume a substantially straight configuration.

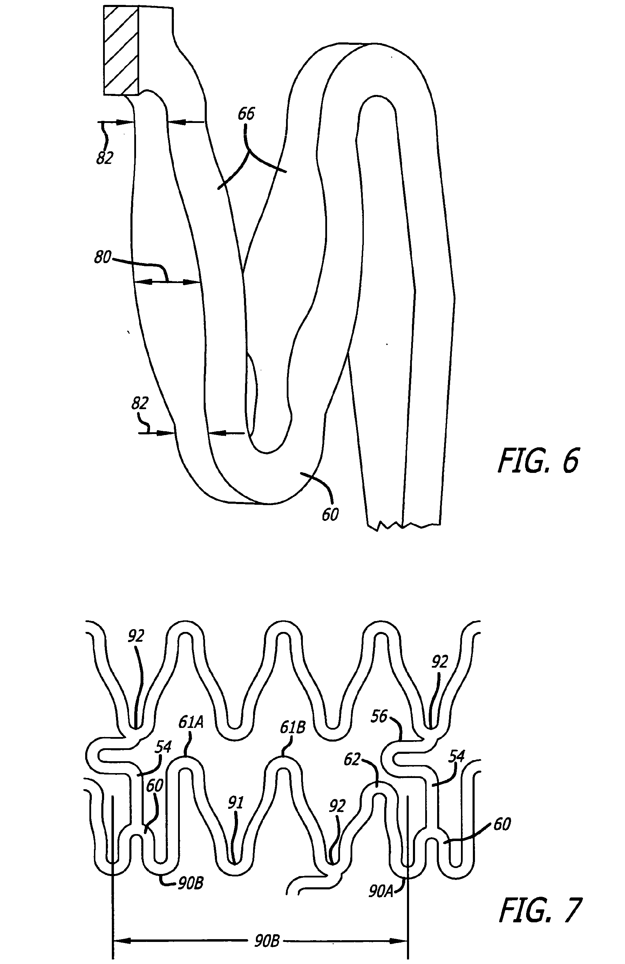

In another aspect of the invention, as shown in FIG. 6, the stent 30 is formed so that the various elements of the cylindrical rings 40, including the long struts 67, short struts 66, intermediate struts 68, various peaks 60, 61, 62, various valleys 90, 91, 92, and the undulating links 54, all can be formed so that each has a variable thickness along the stent length, For example, the undulating link 54 may be thicker at the arm 76 portion than at the extension 78 portion of the link. Such structure can reduce deployment pressure while maintaining radial strength. Further, short struts 66, long struts 67, and intermediate struts 68 may vary in thickness (radial thickness) along their length in order to create variable flexibility in the rings. Moreover, in one contemplated approach short peak 60 has short struts 66 that can have radial thick portion 80 in the middle of the struts and radial thin portion 82 near the ends of the struts. As another example, (not shown), the ring 40E at the proximal end 46 and/or ring 40 at the distal end 48 of the stent may be thicker radially than the other cylindrical rings 40 of the stent. It is also to be recognized that the proximal end ring 40E can have a unique configuration, one that is different from the remaining rings of the stent 30.

Turning now to FIG. 7, in one contemplated embodiment, at least one cylindrical ring 40 includes a repeating pattern of peaks and valleys. That is, for example, each pattern segment 88 includes in sequence a short peak 60, a shallow valley 90B, a tall peak 61A, a deep valley 91, a tall peak 61B, an intermediate valley 92, an intermediate peak 62, and a shallow valley 90A. The pattern segment may repeat itself as many times as necessary to provide a cylindrical ring of a desired expansion diameter. Also, adjacent cylindrical rings can be connected by one undulating link 54 per pattern segment. For example, an undulating link connects the short peak 60 of one cylindrical ring to the intermediate valley 92 of an adjacent cylindrical ring. Further, there may be more than one undulating link 54 or straight link 84 (FIG. 5) per pattern segment. In other contemplated embodiments, the additional links 54, 84 may connect any of the various types of peaks 60, 61, 62 and valleys 90, 91, 92 to any other or same type of peak or valley. Adjacent cylindrical rings can have the same repeating pattern or may have different repeating patterns from each other.

Referring back now to FIG. 1, in one embodiment, the stent of the present invention includes a repeating pattern segment including four peaks and four valleys, and the short peak 60 of a first ring 40A is linked to and longitudinally aligned with the intermediate valley 92 of an adjacent second ring 40B. The short peak 60 of the second ring 40B is linked to and longitudinally aligned with the intermediate valley 92 of an adjacent third ring 40C. In other words, the pattern of the second ring 40B is rotated from the pattern of the first ring 40A, and the pattern of the third ring 40C is further rotated from the pattern of the second ring 40B.

This rotational pattern results in the short peaks being longitudinally aligned every third cylindrical ring. Such a three ring longitudinal design may then be repeated as desired to add additional length to a stent In at least one embodiment, the most proximal end 46A or most distal end 46B of the stent 30 may have a row 40E of undulations having all the same length struts 66, or 67, or 68.

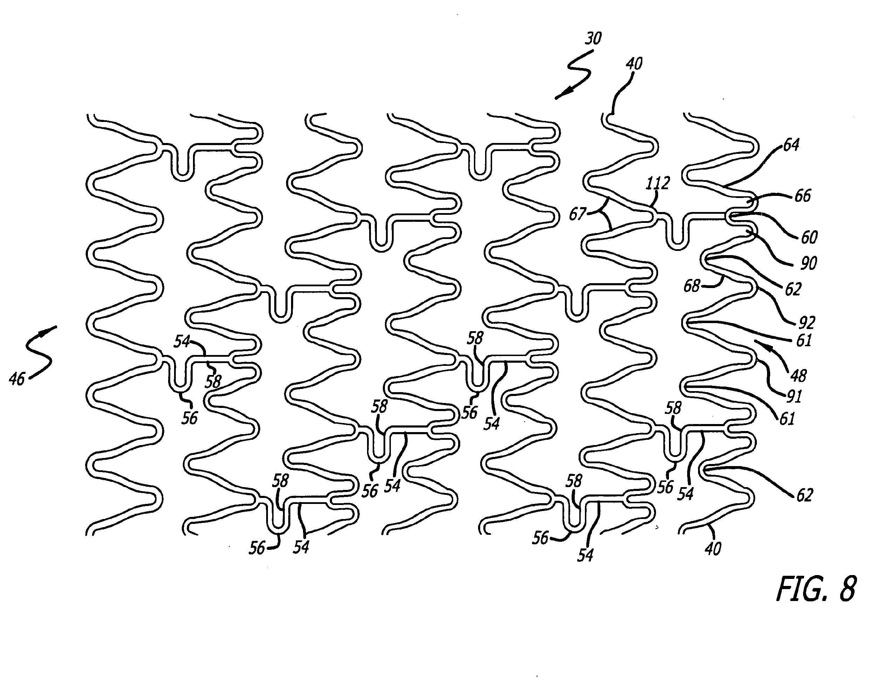

In another design of sheet 30 which includes an open W or butterfly pattern of struts (See FIG. 8), substructures of the stent 30 have been rearranged to provide a pattern which addresses potential flaring of ends 46, 48 of the stent. To accomplish this, long struts 67 defining deep valleys 91 are connected to the links 54 and the struts 67, 68 defining the intermediate valley 92 are positioned circumferentially adjacent thereto. In this way, stresses being released from the W or butterfly structure are transferred to a pair of long struts 67 rather than one long strut 67 and one intermediate strut 68.

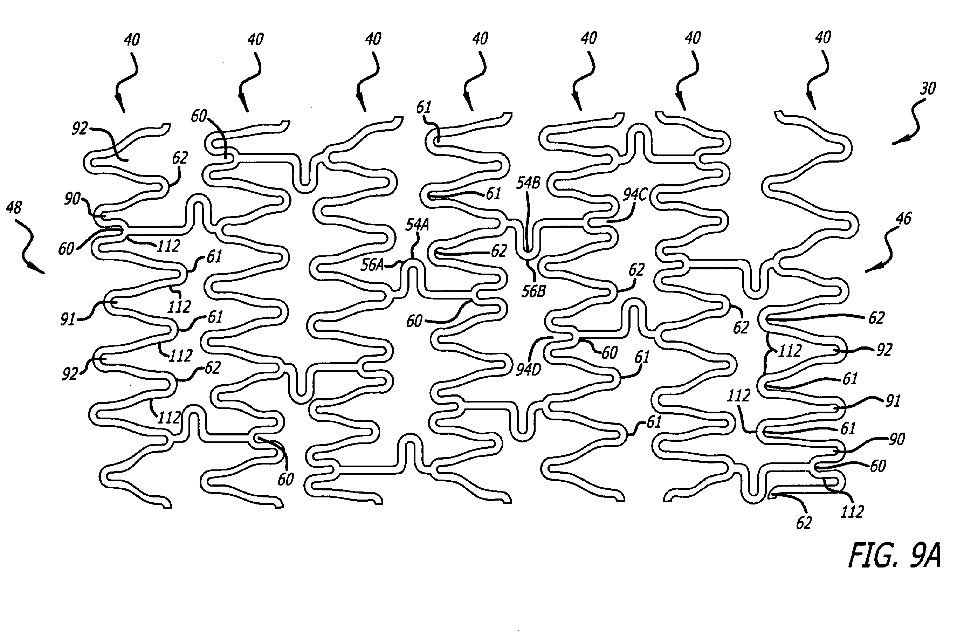

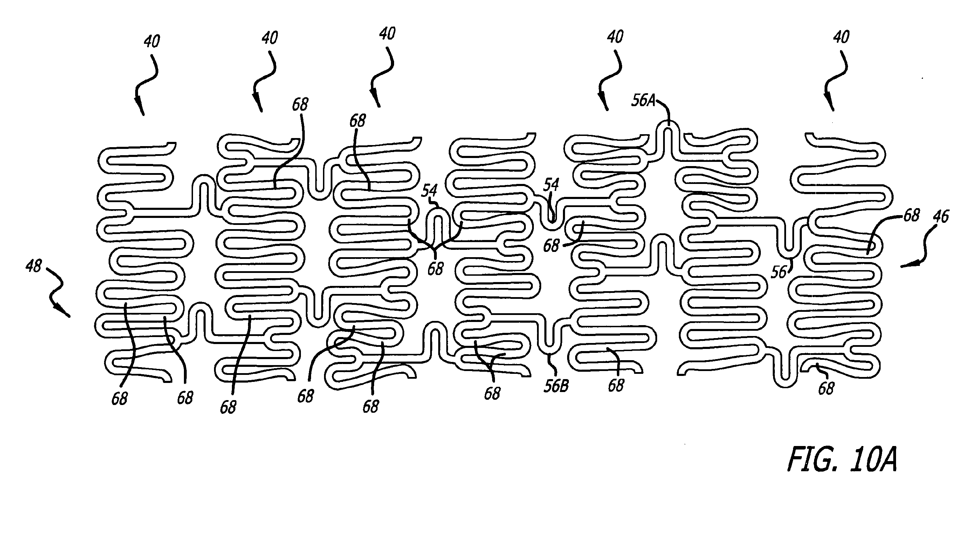

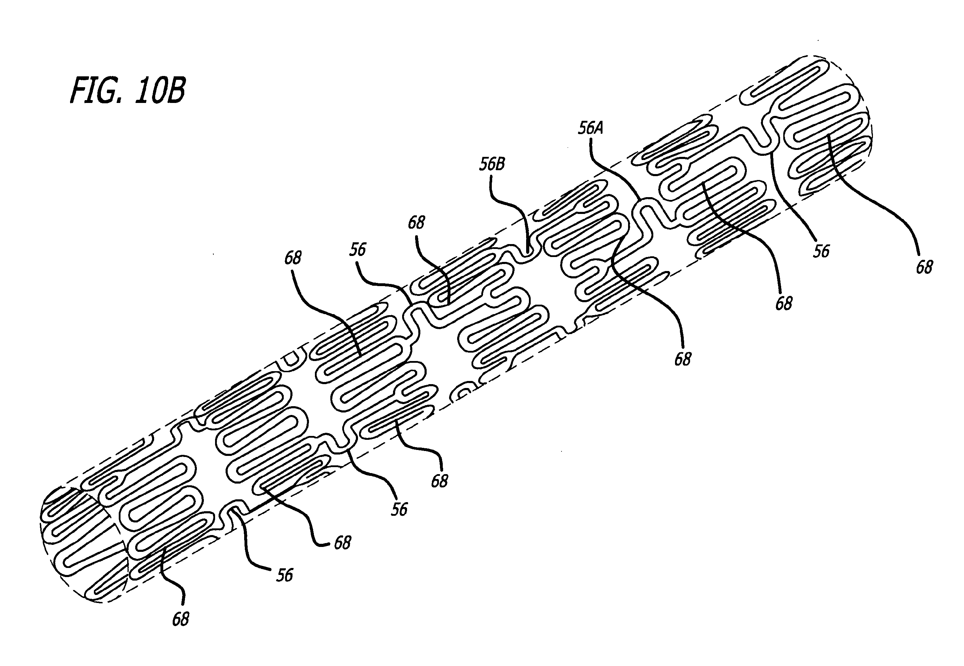

Referring now to FIGS. 9A-10B, yet another aspect of the stent 30 is embodied in a non-directional sent Notably, the non-directional stent may be mounted on a delivery device with either the proximal or distal end of the stent oriented towards the distale end of the delivery device with equal effectiveness. Thus, the non-directional stent may be mounted onto a stent delivery system (not shown), for example a balloon catheter, without it being positioned in a preferred proximal-distal orientation. FIGS. 9A-9B show representative features of a portion of the non-directional stent in an as-manufactured (flat and cylindrical) configuration, and FIGS. 9A-9B show representative features of a portion of the non-directional stent in a compressed configuration (flat and cylindrical).

In one embodiment of the non-directional stent, all of apexes 112 of the peaks 60, 61, 62 on the most proximal end 46 cylindrical ring 40 of the stent 30 point distally and, all of apexes 112 of the peaks 60, 61, 62 on the most distal end 48 cylindrical ring 40 point proximally. Similarly, all of the valleys 90, 91, 92 of the most distal end 48 ring 40 face proximally, and all of the valleys of the most proximal end 46 ring 40 face distally. Stated another way, all of the peaks on the proximal end 46 ring 40 point towards all of the peaks on the distal end 48 ring 40. Additionally, all of the valleys on the proximal end 46 ring 40 point towards all of the valleys on the distal end 48 ring 40.

In a further aspect, along the entire length of the stent, there may be approximately equal numbers of peaks 60, 61, 62 having apexes 112 that point towards the proximal end 46 of the stent 30 and peaks having apexes that point towards the distal end 48 of the stent. There may also be along the entire length of the stent approximately equal numbers of valleys 90, 91, 92 having apexes 112 that point towards the proximal end 46 of the stent 30 and valleys having apexes that point towards the distal end 48 of the stent.

In still another aspect (not shown), it is contemplated that at least some of the intervening rings 40 that are located between the proximal end 46 and the distal end 48 may include peaks 60, 61, 62 having apexes 112 that point proximally, and some of the other rings may include peaks having apexes that point distally. Similarly, at least some of the intervening rings 40 that are located between the proximal end 46 and the distal end 48 may include valleys 90, 91, 92 having apexes 112 that point proximally, and some of the other rings may include valleys having apexes that point distally.

Moreover, in each of the rings 40 of the stent 30 there may be approximately equal number of peaks 60, 61, 62 having apexes 112 that point towards the proximal end 46 of the stent and peaks having apexes that point towards the distal end 48 of the stent. There may also be in each of the rings of the stent approximately equal number of valleys 90, 91, 92 having apexes that point towards the proximal end of the stent and valleys having apexes that point towards the distal end of the stent.

Furthermore, not all of the apexes 112 of the peaks 60, 61, 62 on one ring 40 need to be aligned in the same direction. For example, some of apexes 112 of the peaks 60, 61, 62 on the ring 40 may point distally 48, and some of the apexes of the peaks on the ring 40 may point proximally 46. Also, some of apexes 112 of the valleys 90, 91, 92 on the ring 40 may point distally 48, and some of the apexes of the valleys on the ring 40 may point proximally 46. Additionally, the rings may be rotationally offset from each other or configured to be in-phase.

As illustrated in FIG. 9A, the non-directional stent includes at least two of the butterfly patterns oriented in opposite longitudinal directions from each other. Moreover, in certain approaches, it may be desirable to configure the stent so that the oppositely oriented butterfly pattern may be on the same ring or on different rings, For example, butterfly 94C has an opposite longitudinal orientation from butterfly 94D.

In at least one additional embodiment, not all of the curved portions 56 of the undulating links 54 face in the same orientation in the non-directional stent. For example, as shown in FIGS. 8A-9B, curved portion 56A of undulating link 54A faces in an opposite circumferential direction from curved portion 56B of undulating link 54B.

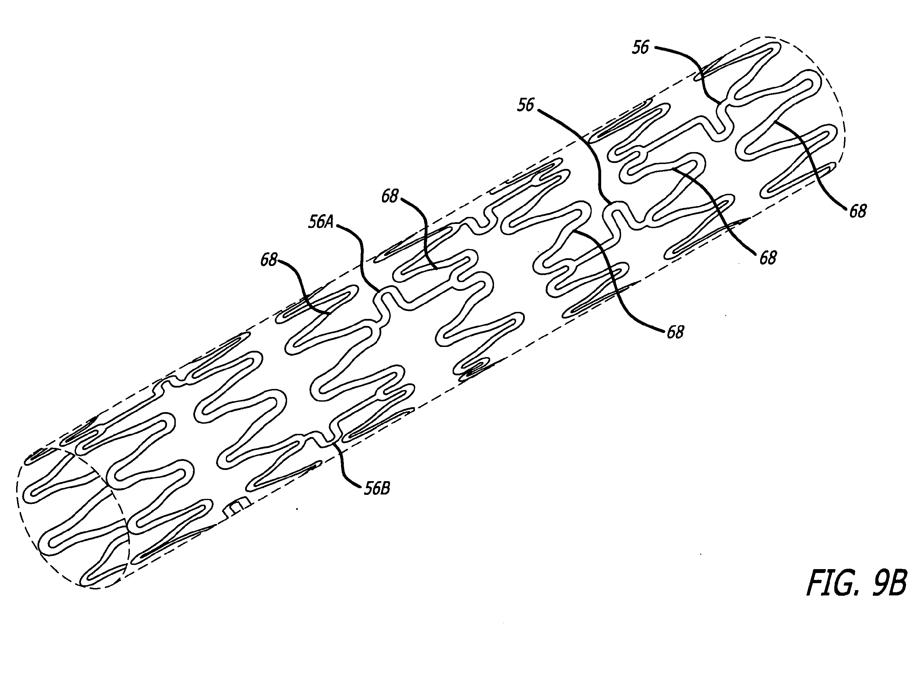

Furthermore, it is also contemplated that a non-directional stent of the present invention can include both the oppositely oriented butterfly patterns of FIG. 9A as well as undulating links facing in opposite circumferential directions as depicted in FIG. 9B. Moreover, such features can be incorporated into directional stents where desired.

Additionally, in the non-directional stent (See FIGS. 9-10B), the intermediate shuts 68 can be configured to remain adjacent to the curved portions 56 of the undulating links 54. Furthermore, the arm 76 of the undulating link 54 is connected to a short peak 60 in the various embodiments of the non-directional stent.

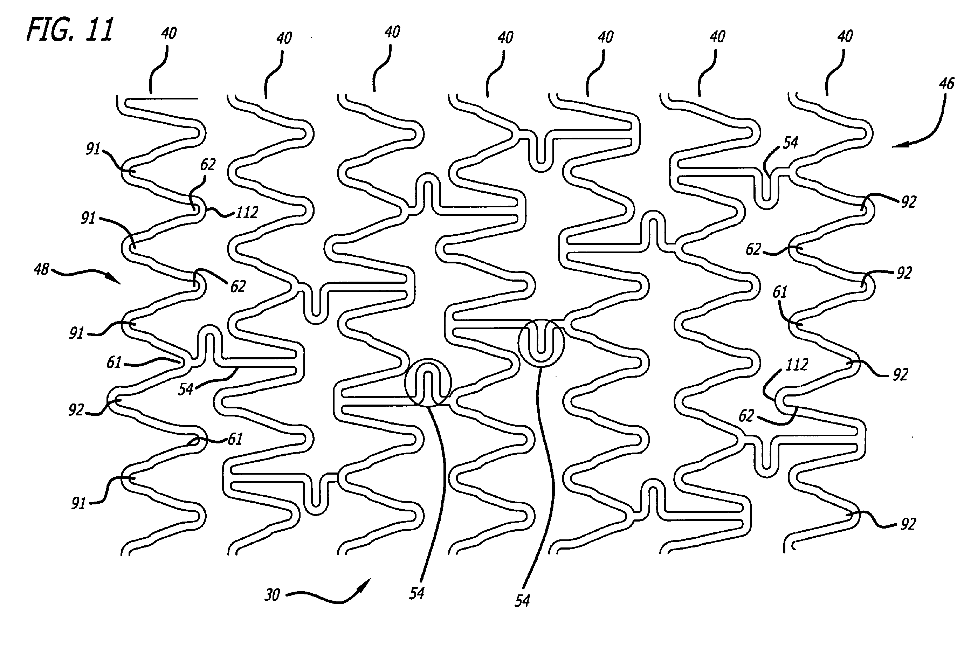

In another design, non-directional stent 30 lacking W or butterfly patterns is also provided (See FIG. 11). That is, a non-directional pattern like that of FIG. 9A is provided except in this design (FIG. 11), the stent 30 does not include the W or butterfly structure. However, this stent 30 can be mounted on a delivery device with either the proximal or distal end of the stent 30 oriented towards the distal end of the stent delivery system. Accordingly, stent 30 includes apexes 112 of the peaks 61, 62 on a proximal end 40 of the stent 30 pointing distally and apexes 112 of the peak 61, 62 on a distal end 48 of the stent pointing proximally. Similarly, the valleys 91, 92 of the most distal end 48 face proximally and the valley 91, 92 of the most proximal leg 46 face distally, As with the previously described approaches, the structure defining the rings 40 and links 54 intermediate the ends 46, 48 of the stent 30 can be configured as desired and thus, can be oriented proximally or distally or in opposite or the same circumferential direction.

Turning now to another aspect of the invention, a "customized" stent may include customized sections that best meet design requirements for desired stent properties. For example, different pattern designs have benefits specific to the particular pattern design. As a non-limiting illustration, the density of drug coating may be varied to meet particular design requirements. A higher coverage pattern may be useful for even drug distribution.

As another illustration of a "customized" stent design, altering the flexibility of the stent in certain regions can improve deliverability. Stents have traditionally been designed with one pattern throughout the length. In accordance with the present invention, however, the flexibility of the pattern can be altered to meet competing design requirements, such as deliverability and drug distribution.

Different patterns can be combined into one stent, or can be used at different portions of the stent, in order to best meet the customer requirements. Alternatively, the thickness and width of the stent strut can be varied (i.e. variable thickness/width) in order to achieve the desired custom design without changing the entire pattern. As a further alternative, different materials can be fused together in order to achieve the custom requirements.

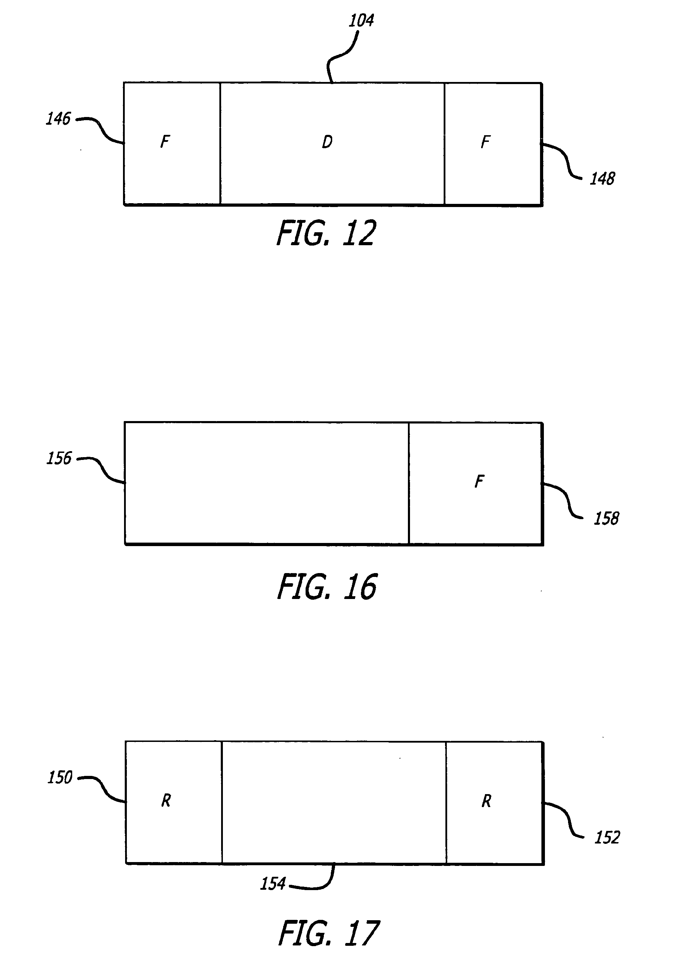

Considering one embodiment of a "customized" stent design, Figure 12 depicts in block form a stent that is more flexible at the end portions than in the center, and that has greater drug coverage in the middle that at the ends. The greater flexibility at the ends improves deliverability when the stent is expanded, and the design minimizes edge injury.

The greater number of links in the center portion minimizes "train-wrecking" and "clam-shell opening" effects of prior art stents when the stent is deployed around a curve. Such curved configurations are often found in the body.

Alternative approaches to making the stent more flexible include making the struts thinner and/or narrower at the ends, as desired. Similarly, the dimensions of the link can be increased, such as by increasing the height and/or width of a "U" shaped link. Also, the number of links connecting the rings may be reduced in order to increase flexibility at desired areas of the stent. Increasing the number of turns in the links also improves flexibility.

With respect to varying the drug coating, one approach is to design the areas of the stent that are to have greater drug coating with more surface area than other areas of the stent.

The surface area in a given area may be increased by, for example, making the struts wider or thicker, or by otherwise increasing the surface area of the stent design, such as by increasing the number of links and/or struts in a given region of the stent. The higher drug coverage in the center portion offers a more even drug distribution by minimizing the high and low dose areas.

Another approach with respect to varying the drug coating on the stent is to employ a method of coating the stent that will vary the amount of drug that is coated at a particular location on the stent. For example, if the stent is coated using a spray method, the spray apparatus may be programmed, as with a numerical control system, to vary the density of coating as a function of location on the stent. This may be done by, for example, varying the spray speed along the length of the stent as the stent is being spray coated. Other known methods in the art for applying a coating in a varied fashion may be employed.

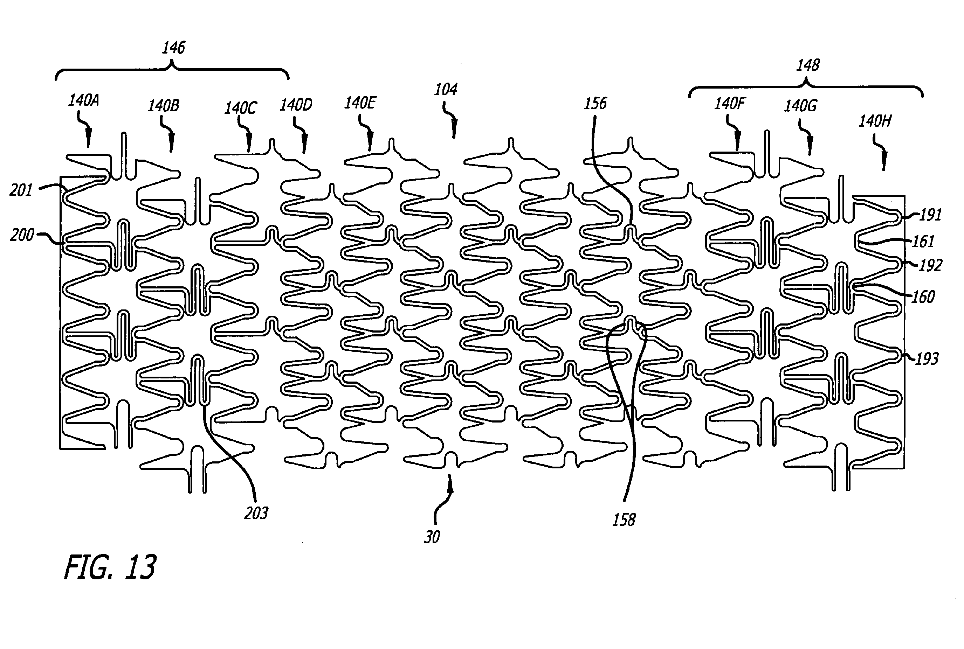

Figures 13-15 depict-one non-limiting exemplary design of this type. This design has more flexible ends and greater drug distribution in the middle. Fig. 13 illustrates a stent having flexible distal and proximal ends, 146 and 148, respectively. The distal end 146 includes three rings, 140A-C. In ring 140A, for example, there are "U"-shaped members 201 that alternate with "M"-shaped members 200. This pattern is repeated in rings 140B and 140C. The rings of the distal end 146 are interconnected with links 203, which have more turns in the curved section.

The proximal end 148 also has rings 140F-H. Ring 140H, at the far distal end, includes a series of relatively "U"-shaped members, as shown. However, it is noted that the valleys 160 and 162 have a somewhat different shape. The valley 160 has a sharper point, whereas the valley 162 is more squared. These relatively sharp valleys 160 alternate with the relatively squared valleys 162, as shown in Fig. 13. Ring 140G is more like the ring 140A, in that it includes "M"-shaped and "U"-shaped members, which is also true of the ring 140F. The rings 140F through 140H are interconnected with links having more turns in the curved section.

The design of the distal end 146 and the design of the proximal end 148 makes the two ends somewhat more flexible than the center portion. The center portion includes rings such as 140D and 140E. The rings have an alternating pattern of links with an open W or butterfly pattern, and links with either straight (not shown) or undulating profile. The rings such as 140D and 140E are interconnected with undulating links.

The net result of the design in Fig. 13 is that the end portions 146 and 148 are more flexible than the center portion. This can be advantageous in the body when, for example, the stent is deployed in a sharp curve within the body.

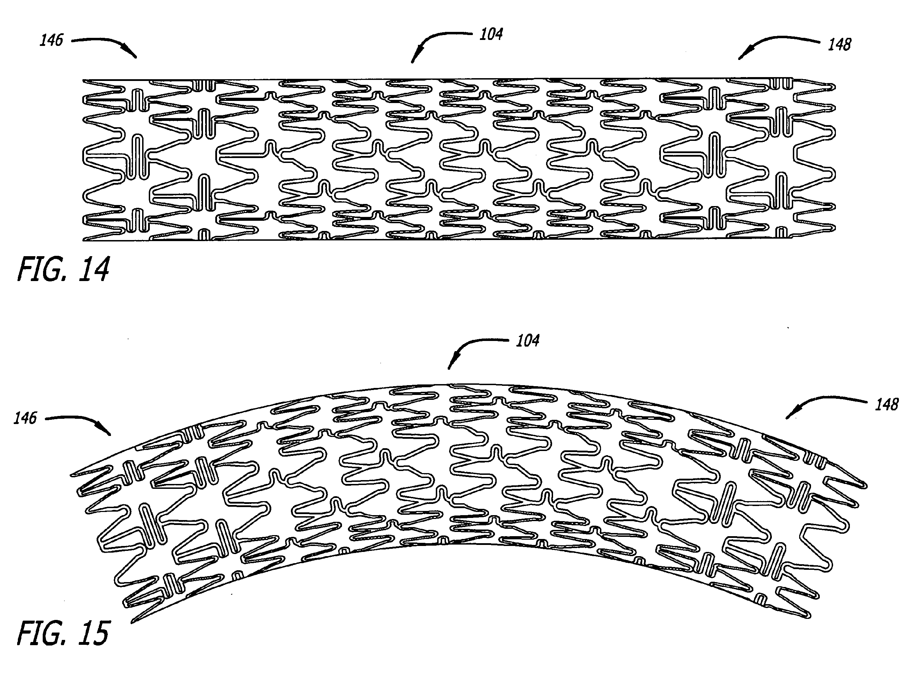

Figure 14 illustrates the stent of Figure 13 as it appears in a deployed state but without bending. The flexible distal and proximal ends are illustrated, as is the center portion, which is less flexible than the distal and proximal ends.

Figure 15 illustrates the stent of Figure 14 as it appears when bent. The center portion, while flexible, is less flexible than either of the ends. As previously noted, the greater flexibility at the ends improves deliverability when the stent is expanded. The design also minimizes edge injury within the vessel.

Another aspect of the stent of Figures 13-15 is the distribution of the drug coating on the stent. The drug coating (not shown) can be concentrated more in the center portion of the stent than on the distal or proximal ends. In the design of Figure 13, for example, the pattern of the struts and links is such that there is greater surface area in the center portion than on the distal or proximal ends. Consequently, the distribution of the drug coating in the center potion is naturally greater in this design than the distribution of the drug coating on the end portions.

Turning now to a further alternative design, Figure 16 depicts in block fashion a design in which a stent has a distal end that is more flexible than one or more other areas of the stent In one embodiment, the distal end is more flexible than the rest of the stent. This is useful for deliverability, since once the distal end gets through a bend the rest of the stent will be more likely to follow through the bend.

To achieve the result of Figure 17, the stent design of Figure 13 may be modified such that the pattern of the center portion 120 is carried out to one of the distal or proximal ends. The remaining end may have the more flexible pattern as shown in Figure 13. Thus, one end will thereby be more flexible. In this case it will be the distal end that is more flexible, although it must be noted that either pattern of end 100 or 102 may be employed to achieve this greater flexibility.

Figure 17 depicts in block-diagram fashion a stent with more radiopaque ends. This can simply be achieved by, for example, designing the end rings to have a greater density of metal than other portions of the stent. The greater density of metal may be achieved by increasing the width and/or thickness of struts, links and/or other portions of the stent. Any other method known in the art for increasing radiopaque properties may be employed to achieve a stent with one or both ends being more radiopaque than other areas of the stent,

The stents of Figs. 12-17 may be used in the same manner as any current stent or drug eluting stent. For example, for arterial stents, the custom stent can be crimped onto a delivery system and delivered through the arteries to a target lesion in the heart. The stent can be deployed by inflating the balloon to the appropriate pressures.

It is noted that this approach can be implemented with stents for various parts of the body, such as with coronary, peripheral, carotid, neuro, and other types of stents. The material can be of stainless steel, CoCr, NiTi, a polymeric stent material, any other material suitable for making stents and known in the art, or any future material developed for stents.

The stents of the present invention can be made in many ways. One method of making the stent is to cut a thin-walled tubular member, such as a stainless steel tubing to remove portions of the tubing in the desired pattern for the stent, leaving relatively untouched the portions of the metallic tubing which are to form the stent. The stent also can be made from other metal alloys such as tantalum, nickel-titanium, cobalt-chromium, titanium, shape memory and superelastic alloys, and the nobel metals such as gold or platinum It is preferred to cut the tubing in the desired pattern by means of a machine-controlled laser as is well known in the art.

Other methods of forming the stent of the present invention can be used, such as using different types of lasers, chemical etching, electric discharge machining, laser cutting a flat sheet and rolling it into a cylinder, and the like, all of which are well known, in the art at this time.

The stent of the present invention also can be made from metal alloys other than stainless steel, such as shape memory alloys, Shape memory alloys are well known and include, but are not limited to, nickel-titanium and nickel-titanium-vanadium, Any of the shape memory alloys can be formed into a tube and laser cut in order to form the pattern of the stent of the present invention. As is well known, the shape memory alloys of the stent of the present invention can include the type having superelastic or thermoelastic martensitic transformation or display stress-induced martensite, These types of alloys are well known in the art and need not be further described here.

The present invention stent is also ideally suited for drug delivery (i.e., delivery of a therapeutic agent) since it has a uniform surface area which ensures uniform distribution of drugs. Typically, a polymer containing the drug is coated onto the stent of the type disclosed in U.S. Patent Nos. 6,824,559 and 6,783,793.

It is contemplated that the stent 30 of the present invention can be mounted on a stent delivery device or system, for example, a balloon catheter (not shown) similar to those known in the prior art. The stent delivery device includes a distal end for mounting of a stent thereon, and a proximal end configured to remain external to a patient's blood vessel. An example of a stent delivery system is disclosed in U.S. Patent No. 6,629,994 entitled "INTRAVASCULAR STENT" filed June 11, 2001. The present invention, however, is not intended to be limited to delivery using the disclosed stent delivery systems but may be used with other stent delivery systems known in the art. The stent may be tightly compressed or crimped on the balloon portion of the catheter and remains tightly crimped on the balloon during delivery through the patient's vascular system. When the balloon is inflated, the stent expands radially outwardly into contact with the body lumen, for example, a coronary artery, When the balloon portion of the catheter is deflated, the catheter system is withdrawn from the patient, and the stent remains implanted in the artery.

Similarly, if the stent of the present invention is made from a self-expanding metal alloy, such as nickel-titanium or the like, the stent may be compressed onto a catheter, and a sheath (not shown) is placed over the stent to hold it in place until the stent is ready to be implanted in the patient. Such sheaths are well known in the art. Once the stent has been positioned at the intended location, the sheath is reacted and the stent self-expands into contact with the wall of the artery. Catheters for delivering self-expanding stents are well known in the art.

It is to be recognized that the invention may be embodied in other forms without departure from the essential charateristics thereof The embodiments described therefore are to be considered in all respects as illustrative and not restrictive. Although the present invention has been described in terms of certain preferred embodiments, other embodiments that are apparent to those of ordinary skill in the art are also within the scope of the invention. Accordingly, the scope of the invention is intended to be defined only by reference to the appended claims.

## Claims

1. A flexible intravascular stent (30) for use in a body lumen, comprising:
a plurality of cylindrical rings (40) aligned along a common longitudinal axis; and
at least one link (54) connecting adjacent cylindrical rings (40);
wherein at least one cylindrical ring (40) includes an open W-pattern, the open W-pattern being connected to no more than one adjacent ring (40);
each of the cylindrical rings (40) have a plurality of first peaks (61), second peaks (62), and third peaks (60), each of the peaks (60, 61, 82) having a height and an apex, the first peaks (61) being taller than the second peaks (62), and the second peaks (62) being taller then the third peaks (60);
each of the cylindrical rings (40) have a plurality of first valleys (91), second valleys (92), and third valleys (90), each of the valleys (90, 91, 92) having a depth and an apex, the first valleys (91) being deeper than the second valleys (92), and the second valleys (92) being deeper than the third valleys (90); and
the third peaks (60) of at least one of the cylindrical rings (40) is connected with the second valleys (92) of an adjacent cylindrical ring (40) by the links (54).

2. The stent of claim 1, wherein the stent (30) includes a plurality of links (54), at least one of the links (54) being an undulating link, the undulating link (54) having a curved portion (56) extending transverse to the stent longitudinal axis towards the second peak (62) and an arm (76) being straight and parallel to a longitudinal axis of the stent (30).

3. The stent of claim 2, wherein the at least one undulating link (54) further includes at least one substantially straight portion (58) connected to the curved portion (56), the at least one substantially straight portion (58) being substantially perpendicular to the stent longitudinal axis,

4. The stent of claim 3, wherein the at least one substantially straight portion (58) of the at least one undulating link (54) is perpendicular to the stent longitudinal axis.

5. The stent of claim 1, wherein the apexes of the first peaks (61) have a first radius (71), and the apexes of the second peaks (62) have a second radius (72), the second radius (72) being different than the first radius (71).

6. The stent of claim 1, wherein the cylindrical ring (40) includes a plurality of a first strut (67), a second strut (68), and a third strut (66), the first struts (67) being longer than the second struts (68), and the second struts (68) being longer than the third struts (66).

7. The stent of claim 6, wherein the open W-pattern is defined by a pair of third struts (66), one first strut (67) and one second strut (68).

8. The stent of claim 6, wherein at least one of the second valleys (92) includes a second strut (68) connected with another second strut (68) by the apex.

9. The stent of claim 6. wherein at least one of the second struts (68) is of dissimilar length than at least one other of the second struts (68).

10. The stent of claim 6, wherein each of the second peaks (62) includes a second strut (68) connected with another second strut (68) by the apex which defines that second peak (62).

11. The stent of claim 1, wherein the stent (30) has a proximal end (46) and a distal end (48) and defines a first longitudinal orientation of the apexes of the peaks (60, 61, 62) and the apexes of the valleys (90, 91, 92) at the proximal end (46) which is generally reversed from a second longitudinal orientation of the apexes of the peaks (60, 61, 62) and the apexes of the valleys (90, 91, 92) at the distal end (48).

12. The stent of claim 1, wherein the stent (30) has a proximal end (46) and a distal end (48) and the apex of at least one third peak (60) of one of the cylindrical rings (40) is oriented towards the proximal end (46) of the stent (30), and the apex of at least one other third peak (60) on another of the cylindrical rings (40) is oriented towards the distal end (48) of the stent (30).

13. The stent of claim 1, wherein the apex of at least one third peak (60) on one of the cylindrical rings (40) is oriented in the opposite direction of the apex of at least one other third peak (60) on the same cylindrical ring (40).

14. The stent of claim 1, wherein orientations of at least some of the peaks (60,61, 62) and some of the valleys (90, 91, 92) are longitudinally reversed along the length of the stent (30) such that the stent (30) may be effectively mounted onto a stent delivery system in at least one of two different orientations.

15. The stent of claim 1, wherein the stent (30) includes at least two undulating links (54), and wherein the curved portion (56A) of at least one of the undulating links (54A) is oriented in an opposite circumferential orientation than the curved portion (56B) of the other undulating link (54B).

16. The stent of claim 1, wherein a plurality of struts define the rings (40) and the link (54), at least a portion of one strut having a variable thickness.

17. The stent of claim 1, wherein a plurality of struts define the rings (40) and the link (54), at least a portion of one strut having a variable width.

## Patentansprüche

1. Flexibler intravaskulärer Stent (30) für die Verwendung in einem Körperlumen, umfassend:
eine Mehrzahl zylindrischer Ringe (40), die entlang einer gemeinsamen Längsachse ausgerichtet sind; und
wenigstens eine Verbindung (54), die benachbarte zylindrische Ringe (40) verbindet;
wobei wenigstens ein zylindrischer Ring (40) ein offenes W-Muster umfasst, wobei das offene W-Muster mit nicht mehr als einem benachbarten Ring (40) verbunden ist;
wobei jeder der zylindrischen Ringe (40) eine Mehrzahl von ersten Gipfeln (61), zweiten Gipfeln (62) und dritten Gipfeln (60) aufweist, wobei jeder der Gipfel (60, 61, 62) eine Höhe und einen Scheitelpunkt aufweist, wobei die ersten Gipfel (61) höher sind als die zweiten Gipfel (62) und die zweiten Gipfel (62) höher sind als die dritten Gipfel (60);
wobei jeder der zylindrischen Ringe (40) eine Mehrzahl von ersten Tälern (91), zweiten Tälern (92) und dritten Tälern (90) aufweist, wobei jedes der Täler (90, 91, 92) eine Tiefe und einen Scheitelpunkt aufweist, wobei die ersten Täler (91) tiefer sind als die zweiten Täler (92) und die zweiten Täler (92) tiefer sind als die dritten Täler (90); und
wobei die dritten Gipfel (60) wenigstens eines der zylindrischen Ringe (40) mit den zweiten Tälern (92) eines benachbarten zylindrischen Rings (40) durch die Verbindungen (54) verbunden ist.

2. Stent nach Anspruch 1, wobei der Stent (30) eine Mehrzahl von Verbindungen (54) umfasst, wobei wenigstens eine der Verbindungen (54) eine wellenförmige Verbindung ist, wobei die wellenförmige Verbindung (54) einen gekrümmten Abschnitt (56) umfasst, der sich quer zu der Längsachse des Stents zu dem zweiten Gipfel (62) erstreckt, und einen Arm (76), der gerade und parallel zu der Längsachse des Stents (30) ist.

3. Stent nach Anspruch 2, wobei die wenigstens eine wellenförmige Verbindung (54) ferner wenigstens einen mit dem gekrümmten Abschnitt (56) verbundenen, im wesentlichen geraden Abschnitt (58) umfasst, wobei der wenigstens eine, im Wesentlichen gerade Abschnitt (58) im Wesentlichen orthogonal zu der Längsachse des Stents ist.

4. Stent nach Anspruch 3, wobei der wenigstens eine, im Wesentlichen gerade Abschnitt (58) der wenigstens einen wellenförmigen Verbindung (54) orthogonal zu der Längsachse des Stents ist.

5. Stent nach Anspruch 1, wobei die Scheitelpunkte der ersten Gipfel (61) einen ersten Radius (71) aufweisen und die Scheitelpunkte der zweiten Gipfel (62) einen zweiten Radius (72) aufweisen, wobei sich der zweite Radius (72) von dem ersten Radius (71) unterscheidet.

6. Stent nach Anspruch 1, wobei der zylindrische Ring (40) eine Mehrzahl einer ersten Strebe (67), einer zweiten Strebe (68) und einer dritten Strebe (66) umfasst, wobei die ersten Streben (67) länger sind als die zweiten Streben (68), und wobei die zweiten Streben (68) länger sind als die dritten Streben (66).

7. Stent nach Anspruch 6, wobei das offene W-Muster durch ein Paar dritter Streben (66), eine erste Strebe (67) und eine zweite Strebe (68) definiert ist.

8. Stent nach Anspruch 6, wobei wenigstens eines der zweiten Täler (92) eine zweite Strebe (68) umfasst, die mit einer weiteren zweiten Strebe (68) durch den Scheitelpunkt verbunden ist.

9. Stent nach Anspruch 6, wobei wenigstens eine der zweiten Streben (68) eine andere Länge aufweist als wenigstens eine weitere der zweiten Streben (68).

10. Stent nach Anspruch 6, wobei jeder der zweiten Gipfel (62) eine zweite Strebe (68) umfasst, die mit einer weiteren zweiten Strebe (68) durch den Scheitelpunkt verbunden ist, der diesen zweiten Gipfel (62) definiert.

11. Stent nach Anspruch 1, wobei der Stent (30) ein proximales Ende (46) und ein distales Ende (48) aufweist und eine erste Längsausrichtung der Scheitelpunkte der Gipfel (60, 61, 62) und der Scheitelpunkte der Täler (90, 91, 92) am proximalen Ende (46) definiert, welche in der Regel die entgegengesetzte Ausrichtung einer zweiten Längsausrichtung der Scheitelpunkte der Gipfel (60, 61, 62) und der Scheitelpunkte der Täler (90, 91, 92) am distalen Ende (48) ist.

12. Stent nach Anspruch 1, wobei der Stent (30) ein proximales Ende (46) und ein distales Ende (48) aufweist und der Scheitelpunkt wenigstens eines dritten Gipfels (60) eines der zylindrischen Ringe (40) zu dem proximalen Ende (46) des Stents (30) hin ausgerichtet ist, und der Scheitelpunkt wenigstens eines anderen dritten Gipfels (60) an einem anderen der zylindrischen Ringe (40) zu dem distalen Ende (48) des Stents (30) hin ausgerichtet ist.

13. Stent nach Anspruch 1, wobei der Scheitelpunkt wenigstens eines dritten Gipfels (60) an einem der zylindrischen Ringe (40) in der entgegengesetzten Richtung zum Scheitelpunkt wenigstens eines weiteren dritten Gipfels (60) an demselben zylindrischen Ring (40) ausgerichtet ist.

14. Stent nach Anspruch 1, wobei Ausrichtungen wenigstens einiger der Gipfel (60, 61, 62) und einiger der Täler (90, 91, 92) entlang der Länge des Stents (30) in Längsrichtung entgegengesetzt sind, so dass der Stent (30) in wenigstens einer von zwei unterschiedlichen Ausrichtungen wirksam an einem Stenteinbringungssystem angebracht werden kann.

15. Stent nach Anspruch 1, wobei der Stent (30) wenigstens zwei wellenförmige Verbindungen (54) umfasst, und wobei der gekrümmte Abschnitt (56A) der wenigstens einen wellenförmigen Verbindung (54A) in einer entgegegengesetzten Umfangsausrichtung zu dem gekrümmten Abschnitt (56B) der anderen wellenförmigen Verbindung (54B) ausgerichtet ist.

16. Stent nach Anspruch 1, wobei eine Mehrzahl von Streben die Ringe (40) und die Verbindung (54) definiert und wenigstens ein Teil einer Strebe eine variable Dicke aufweist.

17. Stent nach Anspruch 1, wobei eine Mehrzahl von Streben (40) und die Verbindung (54) definiert und wenigstens ein Teil einer Strebe eine variable Breite aufweist.

## Revendications

1. Extenseur intravasculaire flexible (30) destiné à être utilisé dans une lumière corporelle, comprenant :
une pluralité d'anneaux cylindriques (40) alignés le long d'un axe longitudinal commun ; et
au moins un lien (54) connectant des anneaux cylindriques adjacents (40) ;
dans lequel au moins un anneau cylindrique (40) comprend un motif en W ouvert, le motif en W ouvert étant connecté à pas plus d'un anneau adjacent (40) ;
chacun des anneaux cylindriques (40) comporte une pluralité de premiers pics (61), de deuxièmes pics (62) et de troisièmes pics (60), chacun des pics (60,61,62) ayant une hauteur et un apex, les premiers pics (61) étant plus grands que les deuxièmes pics (62), et les deuxièmes pics (62) étant plus grands que les troisièmes pics (60) ;
chacun des anneaux cylindriques (40) comporte une pluralité de premiers creux (91), de deuxièmes creux (92) et de troisièmes creux (90), chacun des creux (90,91,92) ayant une profondeur et un apex, les premiers creux (91) étant plus profonds que les deuxièmes creux (92), et les deuxièmes creux (92) étant plus profonds que les troisièmes creux (90) ; et
les troisièmes pics (60) d'au moins un des anneaux cylindriques (40) sont connectés aux deuxièmes creux (92) d'un anneau cylindrique adjacent (40) par les liens (54).

2. Extenseur suivant la revendication 1, ledit extenseur (30) comprenant une pluralité de liens (54), au moins un des liens (54) étant un lien ondulant, le lien ondulant (54) ayant une portion incurvée (56) s'étendant transversalement à l'axe longitudinal de l'extenseur vers le deuxième pic (62) et un bras (76) qui est droit et parallèle à un axe longitudinal de l'extenseur (30).

3. Extenseur suivant la revendication 2, dans lequel ledit au moins un lien ondulant (54) comprend en outre au moins une portion substantiellement droite (58) connectée à la portion incurvée (56), ladite au moins une portion substantiellement droite (58) étant substantiellement perpendiculaire à l'axe longitudinal de l'extenseur.

4. Extenseur suivant la revendication 3, dans lequel ladite au moins une portion substantiellement droite (58) dudit au moins un lien ondulant (54) est perpendiculaire à l'axe longitudinal de l'extenseur.

5. Extenseur suivant la revendication 1, dans lequel les apex des premiers pics (61) ont un premier rayon (71) et les apex des deuxièmes pics (72) ont un deuxième rayon (72), le deuxième rayon (72) étant différent du premier rayon (71).

6. Extenseur suivant la revendication 1, dans lequel l'anneau cylindrique (40) comprend une pluralité d'une première entretoise (67), d'une deuxième entretoise (68) et d'une troisième entretoise (66), les premières entretoises (67) étant plus longues que les deuxièmes entretoises (68) et les deuxièmes entretoises (68) étant plus longues que les troisièmes entretoises (66).

7. Extenseur suivant la revendication 6, dans lequel le motif en W ouvert est défini par une paire de troisièmes entretoises (66), une première entretoise (67) et une deuxième entretoise (68).

8. Extenseur suivant la revendication 6, dans lequel au moins un des deuxièmes creux (92) comprend une deuxième entretoise (68) connectée à une autre deuxième entretoise (68) par l'apex.

9. Extenseur suivant la revendication 6, dans lequel au moins une des deuxièmes entretoises (68) a une longueur différente de celle d'au moins une autre des deuxièmes entretoises (68).

10. Extenseur suivant la revendication 6, dans lequel chacun des deuxièmes pics (62) comprend une deuxième entretoise (68) connectée à une autre deuxième entretoise (68) par l'apex qui définit ce deuxième pic (62).

11. Extenseur suivant la revendication 1, ledit extenseur (30) ayant une extrémité proximale (46) et une extrémité distale (48) et définissant une première orientation longitudinale des apex des pics (60,61,62) et des apex des creux (90,91,92) à l'extrémité proximale (46) qui est généralement inversée par rapport à une deuxième orientation longitudinale des apex des pics (60,61,62) et des apex des creux (90,91,92) à l'extrémité distale (48).

12. Extenseur suivant la revendication 1, ledit extenseur (30) ayant une extrémité proximale (46) et une extrémité distale (48) et l'apex d'au moins un troisième pic (60) d'un des anneaux cylindriques (40) étant orienté vers l'extrémité proximale (46) de l'extenseur (30), et l'apex d'au moins un autre troisième pic (60) sur un autre des anneaux cylindriques (40) étant orienté vers l'extrémité distale (48) de l'extenseur (30).

13. Extenseur suivant la revendication 1, dans lequel l'apex d'au moins un troisième pic (60) sur un des anneaux cylindriques (40) est orienté dans la direction opposée de l'apex d'au moins un autre troisième pic (60) sur le même anneau cylindrique (40).

14. Extenseur suivant la revendication 1, dans lequel les orientations d'au moins certains des pics (60,61,62) et certains des creux (90,91,92) sont inversées longitudinalement le long de la longueur de l'extenseur (30), où l'extenseur (30) peut être monté de manière effective sur un système de distribution d'extenseur dans au moins deux orientations différentes.

15. Extenseur suivant la revendication 1, ledit extenseur (30) comprenant au moins deux liens ondulants (54), et dans lequel la portion incurvée (56A) d'au moins un des liens ondulants (54A) est orientée dans une orientation circonférentielle opposée à celle de la portion incurvée (56B) de l'autre lien ondulant (54B).

16. Extenseur suivant la revendication 1, dans lequel une pluralité d'entretoises définit les anneaux (40) et le lien (54), au moins une portion d'une entretoise ayant une épaisseur variable.

17. Extenseur suivant la revendication 1, dans lequel une pluralité d'entretoises définit les anneaux (40) et le lien (54), au moins une portion d'une entretoise ayant une largeur variable.
